# EUROPEAN PATENT APPLICATION

(11) **EP 4 763 262 A1**
(43) Date of publication of application: **24.06.2026**
(21) Application number: 24222611.6
(22) Date of filing: 20.12.2024
(51) Int. Cl.: A61M 25/10, A61B 17/12

(54) **STOPFLOW CATHETER, ARRANGEMENT AND SYSTEM THEREWITH**

(71) Applicant: Aigner, Karl Reinhard, 84489 Burghausen (DE)
(72) Inventor: Aigner, Karl Reinhard, 84489 Burghausen (DE)
(74) Representative: Schmidt, Christian

(57) **Abstract**

The present disclosure relates to a catheter, an arrangement and a system and methods of using the same. The catheter comprises an occlusion member configured for occluding a blood vessel to limit blood flow through the blood vessel. The catheter is configured to provide a first passage and/or a second passage , wherein the respective passage is configured to provide a connection between an interior of the catheter and an exterior of the catheter within the blood vessel. The first passage is configured to provide a connection between a first lumen of the catheter and the exterior, and/or the second passage is configured to provide a connection between a second lumen of the catheter and the exterior.

## Description

### Background

Treatment of humans or animals with therapeutically active substances or substance combinations is common for many conditions, especially pathological conditions. An undesired side effect when delivering these substances to the blood stream is the distribution of these substances to parts of the body and organism, which are not to be treated. Depending on the concentration of the substance, this may lead to systemic damage. On the other hand, it is often desirable to apply high concentrations of the substances for treating diseases, e.g. tumors, effectively.

Additionally, there are certain treatment methods, e.g. isolated limb perfusion (ILP) or the isolated limb infusion (ILI) for treating patients with solid tumors at the lower limb, which require stopping the blood flow through other body parts, which may be affected by the tumor, as well. For example, ILP and ILI usually require an Esmarch bandage at the thigh and therefore exclude tumor lesions at the pelvic region like frequently occurring lymph node metastases in the groin region or in the, mostly ipsilateral, side of the pelvis.

Hence, there is a need for improved devices and methods for treating humans or animals, and in particular certain target body regions without affecting others.

### Summary

As a general note, the term "patient" as used herein may refer to a human or an animal. The term "distal" is used herein to specify directions, ends or surfaces which are arranged or are to be arranged to face or point towards an end of the catheter which is outside of the patient's body, when the catheter is placed in the patient's body, e.g. in a blood vessel. On the other hand, "proximal" is used to specify directions, ends or surfaces which are arranged or are to be arranged to face or point towards an end of the catheter, which is inside the patient's body, when the catheter is placed in the patient's body. The terms "axial" and "longitudinal" may be used synonymously herein.

The disclosure relates to a catheter. Further, the disclosure relates to an arrangement comprising two catheters and a system for treatment of a patient, e.g. treatment of a pathological condition of the patient. According to the present disclosure, the system may comprise the arrangement. Further, the disclosure relates to a method of using the arrangement or the system for treatment of a patient.

According to an aspect of the disclosure a catheter is provided. The catheter may be a stopflow catheter. The catheter is configured to be placed in the body of a patient, such that at least a proximal end of the catheter is inside the body of the patient. For example, the catheter may be configured to be placed inside a blood vessel of the patient. The catheter comprises an occlusion member, e.g. a balloon or a cage with a membrane. The occlusion member is configured for occluding a blood vessel to limit (i.e. reduce or prevent) blood flow through the blood vessel. For example, when the catheter is placed in the blood vessel and the occlusion member is expanded the natural blood flow (circulation) through a region of the blood vessel behind the catheter is limited. The occlusion member may be operable (expandable and collapsible) for facilitating the positioning of the catheter in the blood vessel and its removal therefrom. The catheter is configured to provide at least one passage, e.g. a first passage. The first passage may be arranged on a first side of the occlusion member, e.g. proximal to the occlusion member. Alternatively, or additionally, the catheter may be configured to provide a second passage. The second passage may be arranged on the first side or on a second side of the occlusion member, e.g. distal to the occlusion member. The first passage and/or the second passage are configured to provide a connection between an interior of the catheter and an exterior of the catheter within the blood vessel, when the catheter is placed in the blood vessel.

The first passage may be configured to provide a connection between a first lumen of the catheter and the exterior. The second passage may be configured to provide a connection between a second lumen of the catheter and the exterior.

In one embodiment, the catheter may be a multi-lumen catheter.

In one embodiment, the occlusion member may be positioned between the first passage and the second passage in an extension direction of the catheter. The extension direction may correspond to a longitudinal axis of the catheter, i.e. a distal-proximal direction. In other words, the occlusion member may be positioned between the first passage and the second passage when seen along the extension of the catheter from a first end of the catheter to a second end of the catheter. For example, the first end may be a proximal end of the catheter, which is or is to be placed inside the blood vessel and the second end may be a distal end of the catheter, which is outside of the patient's body.

In one embodiment, the first lumen may be configured to receive a guide wire, e.g. for guiding the catheter through the blood vessel, during placement of the catheter in the blood vessel. In other words, the first lumen may form a guidewire channel. The first lumen may further be configured such that material, e.g. a fluid, may be delivered into and/or removed from the blood vessel via the first lumen. The fluid may be a body fluid, e.g. blood, a drug (agent, substance, medicament) for treatment of the patient, a mixture thereof, e.g. a drug-blood-mixture, or the like. The material may further comprise non-fluidic materials, e.g. particles of (organic) tissue. The second lumen may be configured such that material, e.g. a fluid, may be delivered into and/or removed from the blood vessel via the second lumen. In other words, the second lumen may form a perfusion channel, especially for preparing and performing isolated perfusion, as described in detail below.

In one embodiment, the second lumen may be configured, e.g. sized and/or shaped, such that a flow rate of at least 100ml per minute, e.g. at least 200ml per minute, at least 250ml per minute, at least 300ml per minute, at least 350ml per minute, at least 400ml per minute or even higher flow rates can be realized therethrough without causing damage to the second lumen and/or the catheter.

In one embodiment, the second passage may be positioned distally to the occlusion member. The second passage may be arranged and designed to reduce turbulence in a region close to the occlusion member, e.g. distal to the occlusion member. Moreover, the second passage may be configured to improve the quality of drug delivery as compared to conventional catheter outlets. For example the second passage according to the present disclosure may enable a more homogenous drug delivery.

The second passage may comprise at least one opening. The opening may be a cut-out in a sidewall of the catheter. In other words, a center axis of the opening may be perpendicular to the longitudinal axis of the catheter. A cross-section of the at least one opening may be round or oval. The diameter of the opening may be at least 2mm, e.g. at least 2.5mm, at least 3mm, at least 3.5mm or at least 4mm. Alternatively, or additionally, the diameter of the opening may be at most 30mm. If the opening has an oval cross-section, an extension of the opening in a longitudinal direction of the catheter may be larger than an extension of the opening in a circumferential direction of the catheter. For example, the diameters of the opening may be at least 2mm and 3mm, e.g. at least 5mm and 4mm, respectively. The diameter in the circumferential direction may be measured as a curve following the circumference or, alternatively, as a direct line between the edges, e.g. between points on opposing edges where a tangent is parallel to the longitudinal axis. The diameter in the longitudinal direction may be the distance between the most proximal and the most distal point of the opening. Alternatively, the opening may have a rectangular cross-section, e.g. a quadratic cross-section.

In one embodiment, a total cross-sectional area of the one or more openings of the second passage may be at least 20mm², e.g. at least 30mm², e.g. between 30mm² and 150mm², preferably between 30mm² and 60mm², e.g. between 37.7mm² and 58.9mm².

In one embodiment, a distance between the occlusion member and the at least one opening of the second passage in the extension direction may be at least 5mm, e.g. at least 10mm, preferably at least 13mm, at least 14mm, or at least 15mm. Alternatively, or additionally, the distance may be at most 50mm. In other words, a distance between the at least one opening and the occlusion member, e.g. a distal end thereof may be at least 5mm, e.g. at least 10mm, preferably at least 13mm, at least 14mm, or at least 15mm. Alternatively, or additionally, the distance may be at most 50mm. The distance may be measured from the distal end of the occlusion member, i.e. the point where an outer diameter of the occlusion member starts increasing in the proximal direction, to a proximal edge of the opening, in specific the most proximal point thereof.

In one embodiment, the second passage may comprise at least two, preferably at least three, openings arranged distally to the occlusion member. The openings may be arranged along the extension direction with a small or without an angular offset therebetween, e.g. such that it is ensured that the openings are connected with the second lumen. A distance between neighboring openings may be the same in the longitudinal direction. For example, a distance between a first opening and a second opening may be the same as a distance between a second and a third opening. If more than three openings are present, at least three openings may be arranged such that a distance between at least some neighboring openings is the same in the longitudinal direction. In one embodiment, a distance between all neighboring openings may be the same in the longitudinal direction. In other words, the openings may be equidistantly distributed along the extension direction of the catheter. In one embodiment, the distance between neighboring openings is between 5mm and 15mm, e.g. 7.5mm, 8mm, or 8.5mm, in the longitudinal direction. The distance may be measured from a most distal point of an opening that is farther proximal to a most proximal point of an opening that is farther distal. In other words, the distance may be an edge-to-edge distance.

In one embodiment, the distance between the openings in the longitudinal direction may be different.

In one embodiment, the occlusion member is distanced from a proximal end of the catheter. For example, a distance between the occlusion member, e.g. a proximal end thereof, and the proximal end of the catheter may be at least 2mm, e.g. between 3mm and 25mm or between 5mm and 20mm, e.g. 8mm. The distance may be measured from a proximal end of the occlusion member, i.e. the point where the outer diameter of the occlusion member starts increasing in the distal direction, to a proximal end of the catheter, i.e. the most proximal point of the catheter. In one embodiment, the distance may be such that the occlusion member does not overlap the first passage in the longitudinal direction.

In one embodiment, a distance between a proximal end of a proximal fixation and the proximal end of the catheter may be at least 2mm, e.g. between 3mm and 15mm, preferably between 3mm and 5mm, e.g. 4mm. In one embodiment, the distance between the occlusion member, e.g. the proximal end thereof, and a proximal end of the catheter is between 5mm and 20mm, preferably between 5mm and 10mm, e.g. 8mm.

In one embodiment, the occlusion member may comprise contrast markings, e.g. X-ray opaque contrast markings, which may be placed at an outer contour of the occlusion member. In one embodiment, the occlusion member is an expandable balloon. An interior of the balloon may be connected to the exterior of the catheter outside of the blood vessel via a third lumen. The third lumen may be configured to be connectable to a fluid supply, e.g. reservoir containing a fluid, for expanding the balloon. At a proximal end, the third lumen may form a third passage, e.g. an inflation and deflation opening, for connecting the third lumen to an interior of the balloon. Likewise, the balloon may be configured to be collapsible via the third lumen, e.g. by removing the fluid from the balloon via the third passage and the third lumen. The balloon may extend along the extension direction of the catheter by at least 25mm, e.g. by at least 30mm, preferably by at least 33mm. Alternatively, or additionally, the balloon may extend along the extension direction of the catheter by at most 50mm. An outer diameter of the balloon may be at least 25mm, preferably at least 30mm. The balloon may have a capacity of at least 15ml, e.g. between 20ml and 30ml, e.g. 25ml.

In one embodiment, a proximal end and/or a distal end of the balloon may be fixed to the catheter, e.g. to its sidewall, via respective fixations, e.g. a proximal fixation and a distal fixation. The fixations may be integrally formed with the balloon, e.g. end portions thereof. In one embodiment, the fixations may extend along the distal-proximal direction by at least 1mm, e.g. by 3mm, 4mm, 5mm, or even more.

In one embodiment, all lumina may extend by the same length in the longitudinal direction. Alternatively, the lumina may have different lengths in the longitudinal direction. For example, the second lumen may end after the second passage. Likewise, the third lumen may not extend beyond the occlusion member in the proximal direction. The third lumen may extend until the proximal end of the catheter.

In one embodiment, the second lumen has a larger cross-sectional area than the first lumen and/or the third lumen. The cross-sectional area may be defined by the diameter, e.g. a minimum diameter, a maximum diameter and/or an average diameter.

For example, the second lumen may have a round cross-section with an internal diameter of at least 1.5mm, e.g. an internal diameter between 1.5mm and 3mm, e.g. between 1.95mm and 2.05mm, preferably 2mm. The first and/or the third lumen may have a round cross-section with an internal diameter of less than 1.5mm, e.g. an internal diameter between 1.05mm and 1.15mm, preferably 1.10mm. A size and/or a shape of the cross-sections of the first lumen and the third lumen may be the same.

In one embodiment, at least one of the first lumen, the second lumen and the third lumen may be spaced from an outer surface of the catheter sidewall, e.g. by at least 0.1mm, preferably by at least 0.2mm. Alternatively, or additionally, a distance between at least one of the first lumen, the second lumen and the third lumen from the outer surface of the catheter sidewall may be less than 4mm, e.g. at most 3mm or at most 2.5mm.

In one embodiment, a distance between the lumina may be at least 0.1mm, preferably at least 0.25mm. Alternatively, or additionally, the distance between the lumina may be equal to or less than 4mm, e.g. equal to or less than 3.5mm, equal to or less than 3.0mm, equal to or less than 2.5mm, equal to or less than 2.0mm, equal to or less than 1.5mm, or equal to or less than 1.0mm. In other words, the lumina may be configured not to contact each other or not to communicate with each other.

In one embodiment, an outer diameter of the catheter may be 12 French. In one embodiment, the outer diameter of the catheter may be at least 3.85mm, e.g. between 3.92 and 4.04mm, preferably 3.97mm. The catheter may have a length, i.e. an extension in the longitudinal direction, of at least 450mm, e.g. at least 500mm, preferably at least 600mm. Alternatively, or additionally, the catheter may have a length of less than or equal to 1000mm.

In one embodiment, the proximal end of the catheter may be configured to provide an atraumatic tip, e.g. a rounded tip, to minimize adverse effects, e.g. on an inner surface of the blood vessel, when the proximal end of the catheter is navigated trough the blood vessel to its desired position.

In one embodiment, the catheter may comprise a connection portion for connecting an interior of at least one of the first lumen, the second lumen and the third lumen to an exterior of the catheter outside the blood vessel. The connection portion may comprise at least one connection element, preferably a connection element for each lumen. The connection element may be configured such that the lumen may be connected to an external device and/or the lumen may be accessed from the outside of the catheter. For example, the connection element may provide an opening for introducing material, e.g. a fluid or a guide wire, into the lumen. In one embodiment, the connection element may comprise a Luer-lock.

In one embodiment, a proximal end of the first lumen may be configured to provide a stop surface for a guide wire. For example, at least a central portion of the proximal end of the first lumen may be closed. The first passage may be arranged with an inclination relative to the longitudinal axis of the catheter. In other words, the first passage may be eccentric with respect to a center of the first lumen and/or inclined relative thereto. For example, the first passage may provide an opening which may be oblique to the extension direction of the catheter. In other words, a center of the opening may not be perpendicular or parallel to the longitudinal axis of the catheter.

The opening of the first passage may be dimensioned like the at least one opening of the second passage, especially regarding the shape and/or the cross-sectional area.

In one embodiment, the catheter may comprise at least one marking, preferably at least five markings. The markings may be distributed in a predetermined pattern along the extension direction of the catheter. A distance between the markings may be between 5mm and 500mm, depending on the length of the catheter. For example, the distance between the markings may be at least 5mm, e.g. at least 10mm, at least 15mm, at least 20mm, at least 30mm, at least 40mm, at least 50mm, at least 60mm, at least 70mm, at least 80mm, at least 90mm, at least 100mm, at least 110mm, at least 120mm, at least 130mm, at least 140mm, at least 150mm, or even more. A most proximal marking may be offset from the proximal end of the catheter by at least one of these distances, as well. The markings may be configured to facilitate the correct positioning of the catheter in the blood vessel. For example the markings may comprise an X-ray opaque material. Alternatively, or additionally, the markings may comprise any other material which is recognizable by common imaging methods.

In one embodiment, the markings may be different from each other. For example, a first marking may comprise one circumferential line indicating not only a position of the first marking relative to the proximal end of the catheter but also that it is the first marking. The other markings may be designed and arranged in similar fashion. By way of example, a second marking may comprise two circumferential lines, a third marking may comprise three circumferential lines, a fourth marking may comprise four circumferential lines, and so on.

The material of the catheter may be biocompatible. For example, the material of the catheter may comprise one or more of polyethylene, polypropylene, polyvinylchloride, polyurethanes, polycarbonates, polyamide and silicone elastomers.

According to an aspect of the disclosure an arrangement (kit) is provided. The arrangement comprises two catheters. At least one of the catheters, e.g. both catheters, may be a catheter as described herein. In one embodiment, the two catheters may have similar characteristics, e.g. the same characteristics, regarding their materials, features and/or functions. For the sake of facilitating the explanations, the following will refer to the "first" catheter as the catheter which is positioned before the target body region in the natural blood flow of the patient, while the "second" catheter will be the catheter, which is placed behind the target body region in the natural blood flow. At least the first catheter may be a catheter as described herein. The second catheter may be a catheter as described herein. Alternatively, the second catheter may comprise at least an occlusion member and a passage distal to the occlusion member, wherein the passage is configured to provide a connection between an interior of the second catheter and an exterior of the second catheter within the blood vessel.

In one embodiment, a target body region between the two catheters placed in the patient's body, may be isolated from the rest of the blood circulation of the patient by the occlusion members of the first and the second catheter, i.e. when the occlusion members are operated (expanded).

In one embodiment, the two catheters may be configured differently. For example, a first catheter may be configured to be placed inside an arterial blood vessel and a second catheter may be configured to be placed inside a venous blood vessel, e.g. a corresponding venous blood vessel. Hence, due to the higher blood pressure in arterial blood vessels, the first catheter may be stiffer than the second catheter, while maintaining sufficient flexibility for a smooth insertion in the blood vessel.

In one embodiment, the catheters may be configured to be placed inside the same blood vessel. Hence, the characteristics of the catheters may be the same.

In one embodiment, the arrangement may comprise at least one tube for connecting at least one of the catheters to an external device, e.g. a pump, and/or a reservoir with a liquid for expanding the balloon. For example, the arrangement may comprise a tube for at least one of the lumina of the catheters, e.g. for each first and/or second and/or third lumen of the first catheter and/or for each first and/or second and/or third lumen the second catheter. In other words, the arrangement may comprise between one and six tubes.

In one embodiment, the arrangement may comprise a filter element. The filter element may be configured to filter molecules from a fluid passing through the filter element, e.g. molecules. The filter element may be configured to filter molecules up to the size of albumin.

The filter element may comprise or may be a hemofilter configured to filter toxic elements, e.g. a toxic agent, from a fluid, e.g. a drug-blood-mixture, passing through the filter element. For example, the hemofilter may be configured to reduce the concentration of the toxic elements in the fluid passing through the filter element, to a concentration which does not cause harm to the organism of the patient.

In one embodiment, the filter element may comprise or may be a chemofilter configured to filter a chemotherapeutic agent (substance) from the fluid passing through the filter element. For example, the chemofilter may be configured to reduce the concentration of the chemotherapeutic agent in the fluid passing through the filter element, to a concentration which does not cause harm to the organism of the patient.

For arriving at a non-harmful concentration, the fluid may need to pass the filter element several times. In other words, the filter element may be configured to prevent systemic damage in the patient's body by filtering toxic elements, e.g. a chemotherapeutic agent, from the drug-blood mixture passing through the filter element. A filtration rate of the filter element may depend on a size of the molecules to be filtered and/or on a substitution volume for replacing the filtered molecules, fluids and/or salts.

In one embodiment, the arrangement comprises at least one, preferably at least two, external flow restriction element. The flow restriction element may be configured to limit a blood flow through a body region, e.g. a limb, when connected to said body region and operated at the body region. For example, the flow restriction element may be a pneumatic cuff, which may be attached to a limb and inflated. By way of example, the arrangement may comprise two pneumatic cuffs configured to be placed on the arms and/or legs of the patient to limit the blood circulation therethrough, when inflated.

According to an aspect of the disclosure a system for treatment of a patient, e.g. a pathological condition of the patient, is provided. The system comprises the arrangement as described herein and a pump. The pump may comprise or may be configured to be connectable to a blood purification unit. The blood purification unit may be configured to receive the filter element.

The pump may be configured to be operatively connectable to or connected to the second lumen of the first catheter and/or the second lumen of the second catheter of the arrangement. The pump may be configured to circulate a fluid between the first catheter and the second catheter. For example, the first catheter may be placed in a first blood vessel, e.g. an artery, the second catheter may be placed in the same or in a second blood vessel, e.g. a corresponding vein. The pump may be configured to circulate a fluid, e.g. a drug, blood and/or a drug-blood-mixture, between the first catheter and the second catheter, thereby establishing a perfusion circulation between the catheters. If the patient's blood circulation is limited or blocked between the two catheters, the pump is configured to operate an isolated perfusion circulation between the catheters.

In one embodiment, the pump may be configured to deliver (or infuse) the fluid into the patient's body, e.g. an arterial blood vessel, via the second lumen and the second passage of the first catheter. Optionally, there may be a first tube for connecting the pump to the second lumen of the first catheter, if the second lumen is not directly connected to an exit portion of the pump. After infusion, the fluid may penetrate a target body region of the patient. As the second catheter may be positioned in a corresponding venous blood vessel, the fluid may arrive at the second catheter, and the pump may be configured to suck the fluid into the second lumen of the second catheter via the second passage of the second catheter. Via the second lumen of the second catheter, and optionally via a second tube, the fluid may arrive at a suction portion of the pump.

In one embodiment, the pump may be configured to filter and/or re-infuse the fluid into the patient's body. Hence, an isolated perfusion circulation may be established between the two catheters via the pump, thereby allowing a penetration of the fluid through the target body region while preventing the fluid from reaching other body regions of the patient.

In one embodiment, according to the desired body region to be treated, both catheters may be placed in the same blood vessel and an isolated perfusion circulation may be established therebetween. This may be beneficial if the desired body region is particularly small.

According to an aspect of the disclosure a method of using the arrangement and/or the system as described herein, is provided. The method may be a method for treatment of a patient, e.g. a desired or target body region. The target body region may comprise organs and/or body tissue which is penetrated by blood vessels.

The method comprises the steps of inserting and positioning the first catheter into a blood vessel of the patient and inserting and positioning the second catheter into the same or another blood vessel of the patient. The catheters may be guided through the blood vessels by visualizing markings, e.g. via X-ray. Additionally, the balloons of the catheters may be filled with a contrast agent.

When correctly positioned, the first and the second catheter may be fixed in the blood vessels at their desired positions. These positions depend on the target body region to be treated. When filled with contrast agent, the balloons may be emptied when the catheters have reached their desired positions.

The method further comprises the step of delivering (infusing) drug to the target region via the first catheter, e.g. via the first and/or the second lumen, and/ or via an additional angiocatheter. The infusion may be constant or in a pulsatile manner for a duration of 30-60 seconds.

In one embodiment, the method may comprise the step of limiting, e.g. blocking, the natural blood circulation of the patient, at least in the target body region, by operating, e.g. expanding, occlusion members of the first and the second catheter. In other words, the blood circulation between the first and the second catheter may be stopped. This state of limited, preferably no, blood circulation between the catheters is called a stopflow phase. The stopflow phase may be maintained for a predetermined time, e.g. at least 30 seconds, at least 1 minute, and preferably up to 5 minutes. During the stopflow phase, the drug may distribute in the target body region.

In one embodiment, a perfusion circulation may be initiated, e.g. after the stopflow phase. During the perfusion circulation, a fluid (as described in the foregoing) may circulate between the two catheters and trough the target body region.

For example, the pump may be operated to pump a fluid into the first catheter so that the fluid exits a second passage of a second lumen of the first catheter. The fluid may penetrate the target body region and may enter a second passage of a second lumen of the second catheter. A suction function of the pump may facilitate the entry of the fluid into the second lumen of the second catheter.

In one embodiment, the perfusion circulation may not require a pump but may be driven by the natural blood flow, depending on the position of the catheters in the patient's body.

In one embodiment, if the occlusion members remain expanded during the perfusion circulation, an isolated perfusion circulation is established, i.e. the circulation happens only through the catheters and the target body region while the fluid does not penetrate other parts of the patient's body. This means that the fluid is delivered via the first catheter into the patient's body, i.e. to the target body region, and then penetrate only the target body region, before exiting the patient's body via the second catheter.

In one embodiment, the step of limiting or blocking the natural blood flow through the target body region may be performed with a delay between operating the occlusion members. For example, an occlusion member of the second catheter may be operated first and only after a predetermined pause, e.g. 30-60 seconds, the occlusion member of the first catheter may be operated for blocking an incoming blood flow. During the predetermined pause, drug may be delivered via the first and/or second lumen of the first catheter and the drug may mix with the incoming natural blood flow. This drug-blood-mixture may penetrate the target body region due to the natural blood flow caused by the incoming blood. During the predetermined pause, the blood pressure of the patient, e.g. in the target region, may be monitored and the length of the pause may be determined based thereon.

After the predetermined pause, the natural blood flow may be interrupted by operating the occlusion member of the first catheter. This may start the stopflow phase, if the perfusion circulation is not immediately initiated.

In one embodiment, the blood circulation through body regions other than the target body region may be further limited by operating the flow restriction element(s), e.g. by inflating pneumatic cuffs. For example, the blood circulation in the limbs may be restricted if the desired body region is not part of the limb.

In one embodiment, the method comprises the step of providing hyperoxygenation to the patient. This may be done before the occlusion member and/or the flow restriction elements are operated. In other words, the patient may receive hyperoxygenation before the natural blood flow is blocked.

In one embodiment, the method may further comprise the step of filtering a substance from the fluid in the (isolated) perfusion circulation, e.g. filtering toxic elements such as a chemotherapeutic agent from the fluid, to reduce the concentration of the substance in the fluid. This step may be performed at the end of the treatment, before the isolated perfusion circulation is stopped and the occlusion members and/or the flow restriction elements are collapsed to reactivate the natural blood circulation of the patient.

In one embodiment, the method may further comprise the step of unblocking the natural blood circulation by operating, e.g. collapsing, the occlusion members of the first and the second catheter. If flow restriction element(s) are used additionally, the step of unblocking the blood circulation includes operating the flow restriction elements, e.g. deflating the pneumatic cuffs. Depending on the concentration of drug in the fluid, the step of unblocking the blood circulation is performed after filtering, only, in order to reduce the impact on the patient's organism.

By way of example, the method according to the disclosure may be one of "Isolated Hypoxic Abdominal Perfusion (HAP)", "Isolated Upper Abdominal Perfusion (UAP)", "Isolated Hypoxic Pelvic Perfusion (HPP)", "Extended Isolated Stopflow Limb Infusion (EISLI)", or "Isolated Thoracic Perfusion (ITP)", as described in detail below.

The aspects of the present disclosure improve the treatment efficacy. For example, the use of higher drug concentrations in the target body regions may be enabled without causing a higher negative impact on the overall organism. This may further lead to shorter treatment times as compared to conventional treatment methods.

Moreover, the catheter according to the present disclosure may reduce turbulence in the patient's blood flow.

Further, by providing the predetermined pause between the operation of the occlusion member of the second catheter and the operation of the occlusion member of the first catheter, the drug-blood-mixture may penetrate the target region better, e.g. more homogenously, thereby improving the treatment efficacy. The efficacy may further be enhanced by the stopflow phase according to the present disclosure.

Lastly, especially in EISLI as described in detail below, the target body region is not limited to the limbs, as compared to the well-known isolated limb perfusion (ILP) or the isolated limb infusion (ILI), but may include the pelvic region, as well. In other words, according to aspects of the present disclosure, it is possible to treat body regions with isolated perfusion, which cannot be treated in an isolated manner according to the state of the art.

The making and using of the presently preferred embodiments are discussed in detail below. It should be appreciated, however, that the present disclosure provides many applicable concepts that can be embodied in a wide variety of specific contexts. The specific embodiments discussed are merely illustrative of specific ways to make and use the disclosed concepts, and do not limit the scope of the claims.

The foregoing has outlined rather broadly the features and technical advantages of embodiments of the present disclosure. Additional features and advantages of embodiments of the present disclosure will be described hereinafter, e.g. of the subject-matter of dependent claims. It should be appreciated by those skilled in the art that the conception and specific embodiments disclosed may be readily utilized as a basis for modifying or designing other structures or processes for realizing concepts which have the same or similar purposes as the concepts specifically discussed herein. It should also be recognized by those skilled in the art that equivalent constructions do not depart from the spirit and scope of the disclosure, such as defined in the appended claims.

### Brief description of the drawings

For a more complete understanding of the presently disclosed concepts and the advantages thereof, reference is now made to the following description in conjunction with the accompanying drawings. Herein, same reference numerals refer to the same technical features if not stated otherwise. The drawings are not drawn to scale. In the drawings the following is illustrated in:
- Figure 1:: a schematic view of a catheter according to an embodiment of the present disclosure,
- Figure 2:: a schematic view of a proximal portion of a catheter according to an embodiment of the present disclosure,
- Figure 3:: a cross-sectional view of a catheter according to an embodiment of the present disclosure,
- Figure 4:: a schematic illustration of an arrangement according to an embodiment of the present disclosure,
- Figure 5:: a schematic illustration of a system according to an embodiment of the present disclosure,
- Figure 6:: a schematic illustration of a perfusion circulation according to an embodiment of the present disclosure, and
- Figure 7:: a schematic illustration of a method of using an arrangement or a system for treatment of a patient according to embodiments of the present disclosure.

### Description of exemplary embodiments

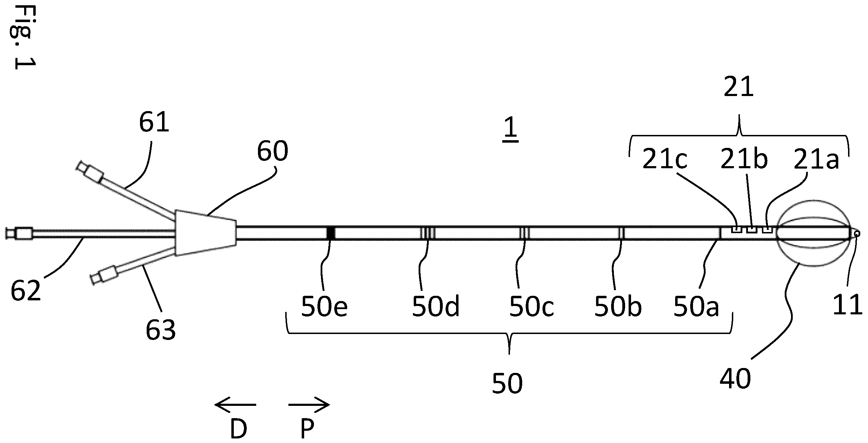

Figure 1 illustrates an embodiment of catheter 1 according to the present invention. The catheter 1 is configured to be placed inside a blood vessel of the patient. The catheter 1 comprises an occlusion member 40, e.g. a balloon 40 as illustrated or a cage with a membrane. The occlusion member 40 is configured for occluding a blood vessel to limit (i.e. reduce or prevent) blood flow through the blood vessel. The occlusion member 40 is operable, i.e. expandable and collapsible, for facilitating the positioning of the catheter in the blood vessel and its removal therefrom. The catheter 1 is configured to provide a first passage 11 on a first side of the occlusion member 40 and a second passage 21 on a second side of the occlusion member 40. In other words, the occlusion member 40 is positioned between the first passage 11 and the second passage 21 in an extension direction of the catheter, i.e. a distal-proximal direction indicated by arrows D and P, respectively.

The first passage 11 and the second passage 21 are configured to provide a connection between an interior of the catheter 1 and an exterior of the catheter within the blood vessel, when the catheter 1 is placed in the blood vessel.

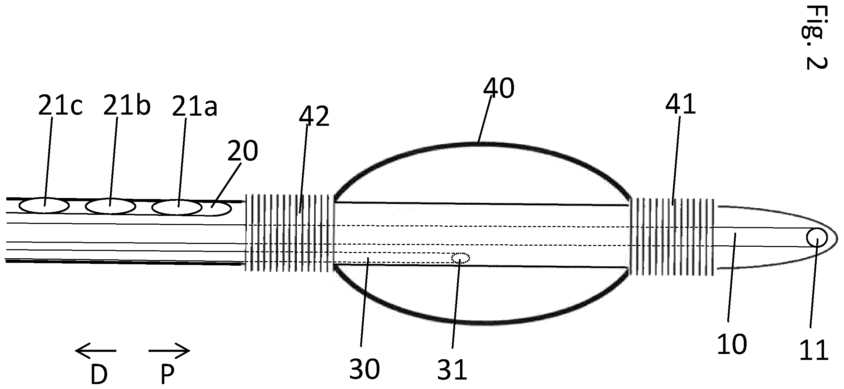

With further reference to Figure 2, the catheter is a multi-lumen catheter. The first passage 11 is configured to provide a connection between a first lumen 10 of the catheter 1 and the exterior. The second passage 21 is configured to provide a connection between a second lumen 20 of the catheter 1 and the exterior. The first lumen 10 forms a guide wire and infusion channel. The second lumen 20 forms a perfusion channel.

The occlusion member 40 may comprise contrast markings (not shown), e.g. X-ray opaque contrast markings, which may be placed on an outer contour. In the illustrated embodiment, the occlusion member 40 is an expandable balloon 40. An interior of the balloon 40 is connected to an exterior of the catheter 1 outside of the blood vessel via a third lumen 30. The third lumen 30 is configured to be connectable to a fluid supply, e.g. a reservoir. For expanding the balloon 40, fluid from the fluid supply may be introduced in the balloon via the third lumen 30 and the opening 31, connected to an interior of the balloon 40. Likewise, the balloon 40 is collapsible by removing the fluid from the balloon 40 via the opening 31 and the third lumen 30.

The balloon 40 extends along the extension direction of the catheter 1 by at least 25mm, e.g. by 33mm. An outer diameter of the balloon 40 is at least 25mm, e.g.30mm. The balloon 40 has a capacity of at least 15ml, e.g. 25ml. As illustrated in Figure 2, a proximal end and a distal end of the balloon 40 are fixed to the catheter, e.g. to its sidewall, via fixations, i.e. a proximal fixation 41 and a distal fixation 42. The fixations 41, 42 may be integrally formed with the balloon 40. The fixations 41, 42 extend in the distal-proximal direction by 4mm.

A proximal end of the catheter 1 is configured to provide an atraumatic tip, e.g. the rounded tip as illustrated. At a distal end, the catheter 1 comprises a connection portion 60 for connecting an interior of at least one of the first lumen 10, the second lumen 20 and the third lumen 30 to an exterior of the catheter 1 outside the blood vessel. The connection portion 60 comprises three connection elements 61, 62 and 63. A first connection element 61 is configured to provide an opening for the first lumen 10 through which a guide wire may be inserted into the first lumen 10. Likewise, a fluid, e.g. a drug, may be introduced in the first lumen 10 via the connection element 61. A second connection element 62 is configured to provide an opening for the second lumen 20, through which a fluid, e.g. a drug, may be introduced in the second lumen 20. A third connection element 63 is configured to provide an opening for the third lumen 30, so that the balloon 40 may be expanded and collapsed by introduction or removal of a fluid, e.g. a contrast agent, respectively. Each of the connection elements 61, 62 and 63 may comprise a Luer-lock.

An outer diameter of the catheter 1 is 12 French or at least 3.85mm, e.g. between 3.92 and 4.04mm, preferably 3.97mm. The catheter 1 has a length, i.e. an extension in the distal-proximal direction, of at least 450mm, e.g. 600mm.

The catheter 1 comprises five markings 50. The markings are distributed in a predetermined pattern along the extension direction of the catheter 1, e.g. every 100mm from the proximal end of the catheter 1, as illustrated. The markings 50 comprise an X-ray opaque material.

Alternatively, or additionally, the markings 50 may comprise any other material which is recognizable by common imaging methods.

The markings 50 are different from each other for indicating not only the position of the catheter but also for identifying the markings. For example, the illustrated first marking 50a comprises one circumferential line indicating that it is the first marking. The other markings 50 are designed and arranged in similar fashion, i.e. the second marking 50b comprises two circumferential lines, the third marking 50c comprises three circumferential lines, the fourth marking 50d comprises four circumferential lines and the fifth marking 50e comprises one broad circumferential line, indicating that it is the last (most distal) marking.

As briefly described before, the first lumen 10 is configured to receive a guide wire for guiding the catheter 1 through the blood vessel, during placement of the catheter 1 in the blood vessel. The first lumen 10 is further configured such that material, e.g. a fluid, may be delivered into and/or removed from the blood vessel via the first lumen 10. The fluid may be a body fluid, e.g. blood, a drug (agent, substance, medicament) for treatment of the patient, a mixture thereof, e.g. a drug-blood-mixture, or the like. The material may further comprise non-fluidic materials, e.g. (organic) tissue. The second lumen 20 is configured to introduce or remove the fluid into or from the patient's body. For example, the second lumen 20 is the main channel for drug infusion and operation of a perfusion circulation.

The second passage 21 comprises three openings 21a, 21b, 21c. The openings 21a, 21b, 21c are lateral openings formed as cut-outs in the sidewall of the catheter 1. A cross-section of the openings is round (not illustrated) or oval. The diameter of the openings may be at least 2mm, e.g. 4mm. The oval openings have an extension in the distal-proximal direction, which is larger than an extension in a circumferential direction of the catheter 1. For example, the diameters of the opening may be 5mm in the distal-proximal direction and 4mm in the circumferential direction, respectively.

The openings 21a, 21b, 21c are arranged in parallel to the extension direction without an angular offset therebetween. The openings 21a, 21b, 21c are equidistantly distributed along the extension direction of the catheter. The distance between neighboring openings is between 5mm and 15mm, preferably between 7.5mm and 8mm in the longitudinal direction, e.g. when measured from edge-to-edge.

A distance between the balloon 40 and the most proximal opening 21a is at least 5mm, e.g. 13mm, 14mm or 15mm. The balloon 40, e.g. a proximal end thereof, is distanced from the proximal end of the catheter 1 by at least 2mm, e.g. by at least 5mm, e.g. by at least 8mm.

In one embodiment, a proximal end of the proximal fixation 41, may be offset from the proximal end of the catheter 1 by at least 3mm, e.g. between 3mm and 5mm, preferably 4mm. The proximal fixation may extend along the distal-proximal direction between 3mm and 5mm, e.g. 4mm.

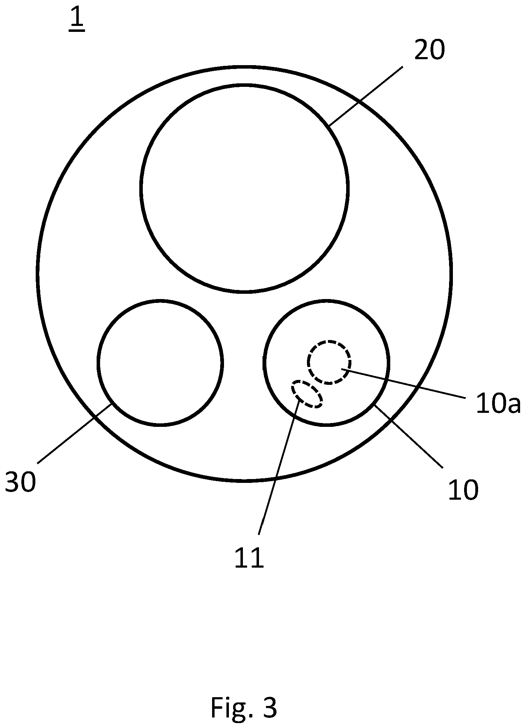

Referring to Figure 3, the second lumen 20 has a larger cross-sectional area than the first lumen 10 and the third lumen 30. For example, the second lumen 20 may have a round cross-section with an internal diameter of at least 1.5mm, e.g. an internal diameter between 1.95mm and 2.05mm, preferably 2mm. The first lumen 10 and/or the third lumen 30 may have a round cross-section with an internal diameter of less than 1.5mm, e.g. an internal diameter between 1.05mm and 1.15mm, preferably 1.10mm. A size and/or a shape of the cross-sections of the first lumen 10 and the third lumen 30 may be the same.

The lumina 10, 20, 30 are spaced from an outer surface of the catheter sidewall by at least 0.1mm, preferably by 0.2mm. A distance between the lumina is at least 0.1mm, preferably 0.25mm.

A proximal end of the first lumen 10 is configured to provide a stop surface for a guide wire. Thus, a central portion 10a of the proximal end of the first lumen 10 is closed. The first passage 11 is arranged with an inclination relative to the longitudinal axis of the catheter 1. Further, the first passage 11 is eccentric with respect to a center of the first lumen 10.

The material of the catheter may be biocompatible. For example, the material of the catheter may comprise one or more of polyethylene, polypropylene, polyvinylchloride, polyurethanes, polycarbonates, polyamide and silicone elastomers.

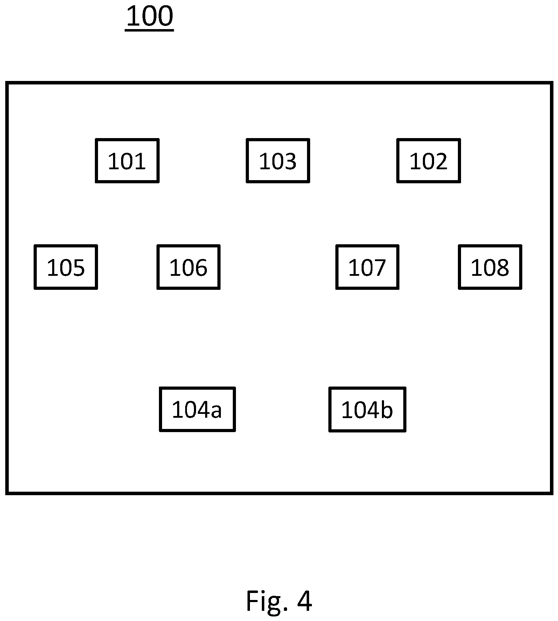

Figure 4 schematically illustrates an embodiment of an arrangement (or kit) 100 according to the present disclosure. The arrangement 100 comprises two catheters 101, 102, which may be two catheters as described herein. The two catheters 101, 102 may have similar characteristics, e.g. the same characteristics, regarding their materials, features and/or functions.

Alternatively, the two catheters 101, 102 may be configured differently. For example, a first catheter 101 may be configured to be placed inside an arterial blood vessel and a second catheter 102 may be configured to be placed inside a venous blood vessel, e.g. a corresponding venous blood vessel. Hence, due to the higher blood pressure in arterial blood vessels, the first catheter 101 may be stiffer than the second catheter 102.

The arrangement comprises four tubes 105, 106, 107, 108 configured for connecting the first catheter 101 and the second catheter 102 to an external device and to a reservoir with a fluid for expanding the balloons 40 of the catheters 101, 102. In specific, a first tube 105 is configured to connect a second lumen of the first catheter 101 to a pump 300 (not shown) and a second tube 106 is configured to connect a third lumen of the first catheter 101 to a reservoir with a fluid for expanding the balloon of the first catheter 101. Likewise, a third tube 107 is configured to connect a second lumen of the second catheter 102 to the pump 300 and a fourth tube 108 is configured to connect a third lumen of the second catheter 102 to a reservoir with a liquid for expanding the balloon of the second catheter 102.

In one embodiment not illustrated, the arrangement 100 additionally comprises a fifth tube and a sixth tube for connecting the first lumina of the first catheter 101 and the second catheter 102 to a drug supply and/or the pump 300, respectively.

The arrangement 100 further comprises a filter element 103. The filter element is configured to filter molecules from a fluid passing through the filter element 103. In one embodiment, the filter element 103 is configured to filter molecules up to the size of albumin. The filter element 103 comprises a hemofilter configured to filter toxic elements from the fluid passing through the filter element 103, e.g. a toxic agent such as a chemotherapeutic agent (substance). The fluid may be a drug-blood-mixture. A filtration rate of the filter element 103 may depend on a size of the molecules to be filtered and/or on a substitution volume for replacing the filtered molecules, fluids and/or salts.

The arrangement 100 comprises two flow restriction elements 104a, 104b. The flow restriction elements 104a, 104b are configured to limit a blood flow through a body region, e.g. a limb, when connected to said body region and operated at the body region. The illustrated flow restriction elements 104a, 104b are pneumatic cuffs, which may be attached to a limb and inflated to stop the blood flow through the limb.

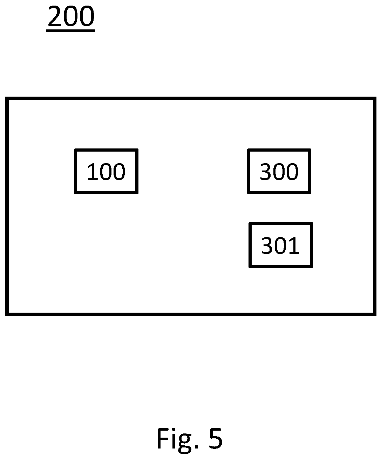

Figure 5 schematically illustrates a system 200 for treatment of a patient, e.g. a pathological condition of the patient. The system 200 comprises the arrangement 100 as described herein and a pump 300. The pump 300 may comprise or may be configured to be connectable to a blood purification unit 301. The blood purification unit 301 is configured to receive the filter element 103 of the arrangement 100.

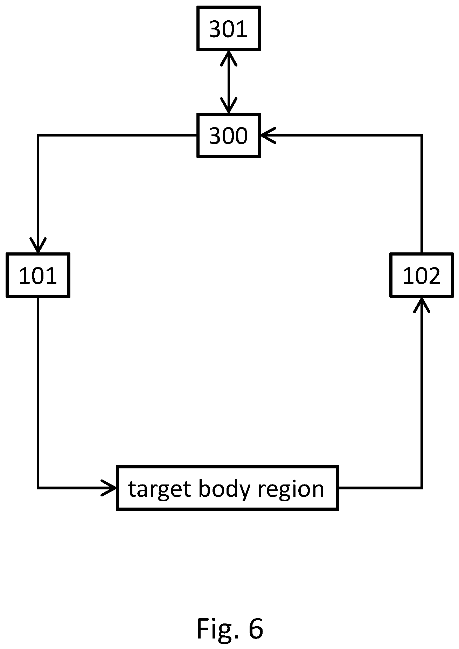

The pump 300 is configured to be connectable to or connected to the second lumina of the first catheter 101 and the second catheter 102 of the arrangement 100. The pump 300 is configured to circulate a fluid between the first catheter 101 and the second catheter 102, thereby establishing a perfusion circulation between the catheters 101, 102. If the patient's blood circulation is limited or blocked between the two catheters 101, 102, the pump 300 is configured to operate an isolated perfusion circulation between the catheters (see Figure 6).

Referring to Figure 6, by way of example, the pump 300 may be configured to deliver (or infuse) the fluid into the patient's body, e.g. an arterial blood vessel, via the first catheter 101. For example, the fluid may be infused via the first lumen and the first passage and/or via the second lumen and the second passage of the first catheter 101. Optionally, there may be the first tube 105 (not shown) and/or another tube for connecting the pump 300, in particular an exit portion thereof, to the second and/or the first lumen of the first catheter 101. After infusion, the fluid may penetrate a target body region of the patient. As the second catheter 102 may be positioned in a corresponding venous blood vessel, the fluid may arrive at the second catheter 102 after having penetrated the target body region. The pump 300 may be configured to suck the fluid into the second catheter 102, e.g. into the second lumen of the second catheter 102 via the second passage. Via the second lumen of the second catheter 102, and optionally via the third tube 107 (not shown), the fluid may arrive at a suction portion of the pump 300. Alternatively, or additionally, a suction portion of the pump may be connected to the first lumen of the second catheter 102 for removing the fluid from the target body region. In one embodiment, the pump 300 may be connected to or include a blood purification unit 301, which is configured to filter the fluid before the fluid is re-infused into the patient's body via the pump 300 and the first catheter 101.

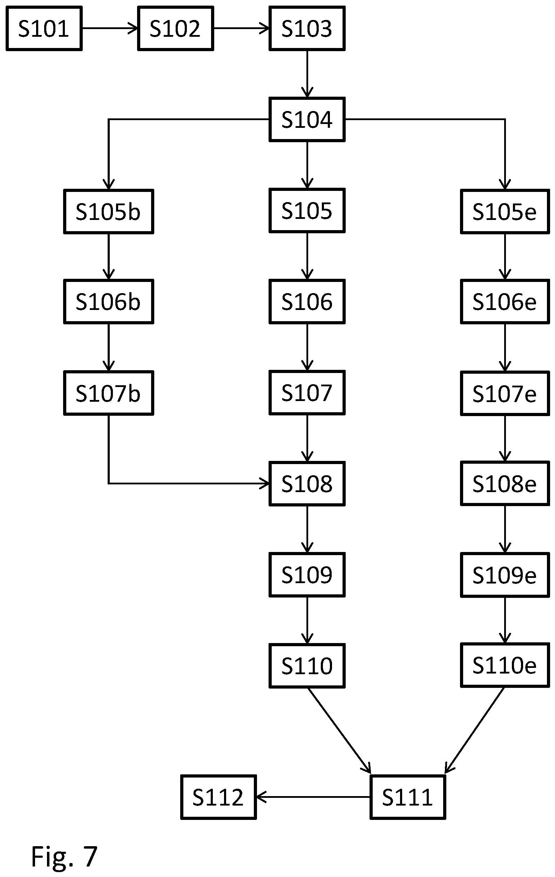

Figure 7 schematically illustrates steps of a method of using the arrangement and/or the system according to the present invention. The method is a method for treatment of a patient, e.g. a desired or target body region. The target body region may comprise organs and/or body tissue which is penetrated by blood vessels.

The method comprises the step, S101, of inserting and positioning two catheters 101, 102 in the patient's body, e.g. in the same or two corresponding blood vessels. The positioning may be guided by markings 50 on the catheters 101, 102 and contrast agent in their balloons 40, which are visible by imaging technologies, e.g. by X-ray.

At their desired positions, the catheters 101, 102 are fixed, S102, and the contrast agent is removed from the balloons 40.

As a next step, hyperoxygenation is provided to the patient, S103, before flow restriction elements 104a, 104b, e.g. pneumatic cuffs, are inflated, S104, to stop the patient's blood flow in body regions which shall not be affected by the treatment.

Afterwards, the method comprises the step of limiting, e.g. blocking, the natural blood circulation of the patient in a region behind the target body region by expanding, S105, an occlusion member of the second catheter 102. In one embodiment, depending on the target body region, an occlusion member of the first catheter may be expanded, S105b, S105e, as will be described in detail with respect to specific treatment methods below.

The method further comprises the step of infusing, S106, drug into the patient's body, e.g. via the second lumen of the first catheter 101. Alternatively, or additionally, the drug may be infused via the first lumen of the first catheter, S106b, S106e, or via an additional angiocatheter, S106, S106e. The infused drug mixes with incoming blood to a drug-blood-mixture whose concentration can be determined according to the desired intensity of the treatment. A length of an optional predetermined pause between drug infusion and expansion, S107, of an occlusion member of the first catheter 101 may determine the concentration of the drug in the drug-blood-mixture. For example, the predetermined pause may be between 30 and 60 seconds.

The method further comprises the step of expanding, S107, an occlusion member of the first catheter, if not already expanded. This may start a stopflow phase in which no fluid circulates in the target body region between the two catheters 101, 102. The stopflow phase may be maintained for a predetermined time, e.g. at least 30 seconds, at least 1 minute, and preferably up to 5 minutes. In UAP treatment, which is described in detail below, the method comprises the step, S107b, of repositioning the first catheter 101 before expanding the occlusion member for starting the stopflow phase.

After the stopflow phase, an (isolated) perfusion circulation is performed, S108, S107e. This may require activating the pump 300 to circulate the fluid, between the catheters 101, 102 and through the target body region. The isolated perfusion circulation may be operated for at least 5 minutes before hemofiltration is started, S109.

In other words, during the isolated perfusion circulation, the pump 300 may be operated to pump a fluid into the first catheter 101 so that the fluid exits a second passage of a second lumen of the first catheter 101. The fluid may penetrate the target body region and may enter a second passage of a second lumen of the second catheter 102. A suction function of the pump may facilitate the entry of the fluid into the second lumen of the second catheter 102.

Depending on the treatment method, the isolated perfusion circulation may be performed for 5 minutes without hemofiltration, followed by 5 minutes with activated hemofiltration (see HAP, UAP, HPP and EISLI, as described below). Afterwards the occlusion members are collapsed and the flow restriction elements are deflated, S110, to re-enable the natural blood circulation.

The hemofiltration continues until a substitution volume of 5 liters is reached, before the pump 300 is switched off, S111. Lastly, the catheters 101, 102 are removed from the patient's body, S112.

In ITP treatment as described in detail below, the isolated perfusion circulation is performed, S107e, by the natural blood flow through the target body region. The isolated perfusion circulation may be performed for 10 minutes without hemofiltration. Then, the occlusion members are collapsed, S108e, and the hemofiltration is started, S109e, by switching on the pump 300. The hemofiltration continues until a substitution volume of 5 liters is reached, before the flow restriction elements are deflated, S110e, and the pump is switched off, S111. However, the steps S110e and S111 may be reversed in ITP treatment, if desired. Lastly, the catheters 101, 102 are removed, S112, from the patient's body.

By way of example, different treatment methods according to the disclosure are described in the following, in more detail.

Isolated Hypoxic Abdominal Perfusion, HAP, is used for treatment of patients with solid cancers in the abdominal region like colon cancer, sarcoma, melanoma, and peritoneal carcinomatosis from any primary tumor.

For HAP, the catheters 101, 102 are inserted, S101, via the groin region into the femoral artery and femoral vein and connected to the external circuit including pump 300 and filtration machine 301. The occlusions members are expandable balloons 40, which are placed in the aorta and the vena cava right beneath the diaphragm. To find the correct placement, the balloons 400 are temporarily filled with contrast media. When the correct placement has been found the catheters 101, 102 are fixed at this position and the balloons are emptied, S102. Then, the patient receives hyperoxygenation, S103, before pneumatic cuffs 104a, 104b at the thighs as well as the balloons 40 are inflated, S104. Between inflation of the venous balloon, S105, and the arterial balloon, S107, there is a short timely shift, i.e. the predetermined pause. The timely shift should not exceed one minute and needs to be surveilled by the anesthetist for maintenance of the thoracic blood pressure. Between the inflation of the venous and the arterial balloon, drug infusion is to be conducted, S106, via the perfusion channel (i.e. the second lumen of the first catheter 101 in the aorta). This is done in terms of a short term infusion with pulsatile infusion over 30-60 seconds to enable a preferable mixture of the drug with the blood from the arterial flow. After the drug infusion and arterial balloon inflation a 5 minute stopflow phase can be conducted during which the highly concentrated drug-blood mixture penetrates the tumor, while the connection to the external circuit remains closed and the pump stays switched off.

After the stopflow phase the isolated perfusion is started, S108, by opening the connection to the external circuit and switching on the pump. During this isolated perfusion phase, the still highly concentrated drug-blood mixture flows through the isolated tumor region (i.e. the target body region) and the connected external circuit. After 5 minutes of isolated perfusion, another 5 minutes of isolated perfusion including the hemofiltration function is performed, S109. After that, in total after 15 minutes of isolation of the tumor region, the balloons 40 and the pneumatic cuffs 104a, 104b are deflated, S110, and the whole body blood circuit is re-enabled. The stopflow catheters remain in the main vessels and hemofiltration is continued until 5 liter substitution volume is reached.

Isolated Upper Abdominal Perfusion, UAP, is used for treatment of patients with pancreatic, liver, gastric, cholangiocellular cancer, liver metastases from any primary cancer and any other solid cancer that is supplied via the celiac trunc.

For UAP, the catheters 101, 102 are inserted, S101, via the groin region into the femoral artery and femoral vein and connected to the external circuit including pump 300 and filtration machine 301. The connection to the external circuit momentarily stays closed. Balloons 40 are placed in the vena cava right beneath the diaphragm and in the aorta right beneath the celiac trunc. To find the correct placement, the balloons are inflated and filled with contrast media momentarily. When the correct placement has been found the catheters 101, 102 are fixed at this positions and the balloons shall be deflated, S102. Then the patient receives hyperoxygenation, S103, before pneumatic cuffs 104a, 104b at the thighs as well as the balloons 40 are inflated, S104. UAP is similar to HAP but has a stopflow phase that is limited to the upper part of the abdomen, that is supplied by the celiac trunc. For this, the stopflow phase has a dynamic arterial balloon placement. The venous balloon is placed beneath the diaphragm and the arterial balloon is placed beneath the celiac trunc. Both balloons are inflated, S105b, whilst the arterial balloon blocks arterial blood flow beneath itself and into the superior mesenteric artery, but allows for arterial blood flow into the celiac trunc and via this into the hepatic artery. Drug infusion is conducted, S106b, in a pulsatile manner via the infusion channel (i.e. the first lumen of the arterial catheter 101) that ends proximal to the tip of the balloon. The arterial blood flow during short term drug infusion over 30-60 seconds enables distribution of the drug in the upper abdominal region. After drug infusion the arterial balloon is quickly pushed upstream above the celiac trunc, S107b, to stop further arterial flow into the tumor region. If necessary, e.g. if the blood vessel is not entirely healthy, e.g. if the patient suffers from arteriosclerosis, the balloon may be partially collapsed, before it is pushed upstream. By way of example, the balloon may be pushed upstream within above the celiac trunc within 2 seconds. However, the speed with which the balloon is pushed upstream may adapted to the condition of the patient's blood vessels, in order to reduce damage of the blood vessel. By pushing the arterial balloon upstream, the stopflow phase is started, as soon as blood flow through the celiac trunc is blocked and highly concentrated drug penetrates the upper abdominal region, while the connection to the external circuit remains closed and the pump stays switched off.

After the stopflow phase the isolated perfusion is started, S108, by opening the connection to the external circuit and switching on the pump 300. During this isolated perfusion phase, the still highly concentrated drug-blood mixture flows through the isolated whole abdominal region and the connected external circuit. After 5 minutes of isolated perfusion, another 5 minutes of isolated perfusion including the hemofiltration function is performed, S109. After that, in total after 15 minutes of isolation of the tumor region, the balloons and the pneumatic cuffs are deflated, S110, and the whole body blood circuit is re-enabled. The stopflow catheters remain in the main vessels and hemofiltration is continued until 5 liter substitution volume is reached.

Isolated Hypoxic Pelvic Perfusion, HPP, is used for patients with solid tumors in the pelvic region like ovarian, cervix, prostate, anal, colorectal, bladder cancer, sarcoma, melanoma, and any other solid cancers in the treatment area.

For HPP, the catheters 101, 102 are inserted, S101, via the groin region into the femoral artery and femoral vein and connected to the external circuit including pump 300 and filtration machine 301. Balloons 40 are placed in the aorta and the vena cava right above the pelvic vessel bifurcation. To find the correct placement, the balloons 40 are inflated and filled with contrast media momentarily. When the correct placement has been found the catheters 101, 102 are fixed at this position and the balloons 40 are deflated, S102. Then the patient receives hyperoxygenation, S103, before pneumatic cuffs 104a, 104b at the thighs as well as the balloons 40 are inflated, S104. For this a short timely shift (predetermined pause) between first the inflation of the venous balloon, S105, and secondly the arterial balloon, S107, can be conducted. The timely shift should not exceed one minute and needs to be surveilled by the anesthetist for maintenance of the thoracic blood pressure. Between the inflation of the venous and the arterial balloon, drug infusion is to be conducted, S106, either via the perfusion channel (i.e. the second lumen) of the arterial catheter 101 or via an additional angiocatheter in one or both iliac artery/arteries. This is done in terms of a short term infusion with pulsatile infusion over 30-60 seconds to enable a preferable mixture with the blood from the arterial flow.

After the drug infusion and balloon inflation a 5 minute stopflow phase can be conducted during which the highly concentrated drug-blood mixture penetrates the tumor, while the connection to the external circuit remains closed and the pump 300 stays switched off. After the stopflow phase the isolated perfusion is started, S108, by opening the connection to the external circuit and switching on the pump. During this isolated perfusion phase, the still highly concentrated drug-blood mixture flows through the isolated tumor region and the connected external circuit. After 5 minutes of isolated perfusion, another 5 minutes of isolated perfusion including the hemofiltration function is performed, S109. After that, in total after 15 minutes of isolation of the tumor region, the balloons 40 and the pneumatic cuffs 104a, 104b are deflated, S110, and the whole body blood circuit is re-enabled. The catheters 101, 102 remain in the main vessels and hemofiltration is continued until 5 liter substitution volume is reached.

Extended Isolated Stopflow Limb Infusion, EISLI, is used as a treatment for patients with solid tumors at the lower limb like melanoma or sarcoma.

For EISLI, the catheters 101, 102 are inserted, S101, via the groin region into the femoral artery and femoral vein and connected to the external circuit including pump 300 and filtration machine 301. Balloons 40 are placed in the aorta and the vena cava right above the pelvic vessel bifurcation for inclusion of the whole pelvic. For sole inclusion of the half of the pelvic that is ipsilateral to the affected limb, the balloon 40 shall be placed at the vessel bifurcation in a way that the blood flow can be stopped between the sides of the pelvis. An additional angiocatheter is inserted, S102, from the healthy limb's groin region into the affected limb and placed ideally in front of the tumor. To find the correct placement, the balloons 40 should be inflated and filled with contrast media momentarily. When the correct placement has been found the catheter is to be fixed at this position and the balloons 40 are deflated, S102. Pneumatic cuffs 104a, 104b are placed at the limb that includes the treatment area below the main tumor region and at the thigh for the other limb. Then the patient receives hyperoxygenation, S103, before pneumatic cuffs 104a, 104b as well as the balloons 40 are inflated, S104. For this a short timely shift (predetermined pause) between first the inflation of the venous balloon, S105, and secondly the arterial balloon, S107, is to be conducted. The timely shift should not exceed one minute and needs to be surveilled by the anesthetist for maintenance of the thoracic blood pressure. Between the inflation of the venous and the arterial balloon, drug infusion is to be conducted, S106, either via the perfusion channel (i.e. the second lumen) of the arterial catheter 101 or via the additional angiocatheter. This is done in terms of a short term infusion with pulsatile infusion over 30-60 seconds to enable a preferable mixture with the blood from the arterial flow.

After the drug infusion and balloon inflation a 5 minute stopflow phase can be conducted during which the highly concentrated drug-blood mixture penetrates the tumor, while the connection to the external circuit remains closed and the pump 300 stays switched off. After the stopflow phase the isolated perfusion is started, S108, by opening the connection to the external circuit and switching on the pump 300. During this isolated perfusion phase, the still highly concentrated drug-blood mixture flows through the isolated tumor region and the connected external circuit. After 5 minutes of isolated perfusion, another 5 minutes of isolated perfusion including the hemofiltration function is performed, S109. After that, in total after 15 minutes of isolation of the tumor region, the balloons 40 and the pneumatic cuffs 104a, 104b are deflated, S110, and the whole body blood circuit is re-enabled. The catheters 101, 102 remain in the main vessels and hemofiltration is continued until 5 liter substitution volume is reached.

Isolated Thoracic Perfusion, ITP, is a treatment for solid tumors in the thoracic region like bronchial carcinoma, breast cancer, mesothelioma, melanoma, lung metastases from any primary tumor and any other kind of solid tumor and metastases that occur in the thoracic region.

For ITP, the catheters 101, 102 are inserted, S101, via the femoral artery and femoral vein in the groin region into the main vessels and connected to the external circuit including pump 300 and filtration machine 301. The connection to the external circuit momentarily stays closed. Balloons 40 are placed in the vena cava and the aorta right beneath the diaphragm. To find the correct placement, the balloons 40 should be inflated and filled with contrast media momentarily. When the correct placement has been found the catheters 101, 102 are fixed at this position and the balloons 40 are deflated, S102. Then the patient receives hyperoxygenation, S103, before pneumatic cuffs 104a, 104b at the upper arms and the balloons 40 are inflated, S104. For ITP both balloons 40 are inflated simultaneously, S105e. The blood flow is continued by the natural heartbeat. Immediately after isolation of the thoracic region by the balloons 40 and the pneumatic cuffs 104a, 104b, the drug is infused, S106e, via the infusion channel (i.e. the first lumen) of the arterial catheter 101 that ends at the tip of the catheter 101, proximal to the balloon 40, or via an additional angiocatheter that can be placed at the tumor supplying artery like the subclavian/mammary artery for breast cancer. 10 minutes of isolated perfusion with a highly concentrated drug-blood-mixture are conducted, S107e, in the isolated thoracic region by the natural heartbeat. After that, the balloons 40 are deflated, S108e, the connection to the pump 300 is opened and hemofiltration is enabled, S109e. Hemofiltration is conducted to the whole body blood volume until a substitution volume of 5 liters is reached. Depending on the desired treatment, the pneumatic cuffs 104a, 104b may be deflated, S110e, before, during or after the pump 300 is operated and hemofiltration is started.

Although embodiments of the present disclosure and their advantages have been described in detail, it should be understood that various changes, substitutions and alterations can be made therein without departing from the spirit and scope of the disclosure as defined by the appended claims.

Further, it should be understood that the embodiments mentioned in the first part of the description may be combined with each other. The embodiments of the description of figures may also be combined with each other. Further, it is possible to combine embodiments mentioned in the first part of the description with examples of the second part of the description which relates to Figures 1 to 7.

### List of reference numbers

- P: proximal direction
- D: distal direction

- 1: catheter
- 10: first lumen
- 10a: central portion of first lumen
- 11: first passage
- 20: second lumen
- 21: second passage
- 21a: first opening
- 21b: second opening
- 21c: third opening
- 30: third lumen
- 31: inflation/deflation opening
- 40: occlusion member
- 41: proximal fixation
- 42: distal fixation
- 50: markings
- 50a: first marking
- 50b: second marking
- 50c: third marking
- 50d: fourth marking
- 50e: fifth marking
- 60: connection portion
- 61: connection element
- 62: connection element
- 63: connection element
- 100: arrangement
- 101: first catheter
- 102: second catheter
- 103: filter element
- 104: flow restriction element
- 104a: first flow restriction element
- 104b: second flow restriction element
- 105: tube
- 106: tube
- 107: tube
- 108: tube
- 200: system
- 300: pump
- 301: blood purification unit

- S101: position catheters
- S102: fix catheters and deflate occlusion members (optionally place angiocatheter)
- S103: hyperoxygenation
- S104: inflate flow restriction elements
- S105: expand second occlusion member
- S105b: expand second occlusion member and first occlusion member at first position
- S105e: expand second and first occlusion member
- S106: infuse drug via second lumen and/or angiocatheter
- S106b: infuse drug via first lumen
- S106e: infuse drug via first lumen and/or angiocatheter
- S107: expand first occlusion member
- S107b: reposition first occlusion member
- S107e: isolated perfusion via natural blood flow
- S108: start/operate isolated perfusion circulation via pump
- S108e: collapse occlusion members
- S109: start/operate hemofiltration
- S109e: activate pump and start hemofiltration
- S110: deflate flow restriction elements and collapse occlusion members
- S110e: deflate flow restriction elements
- S111: switch off pump and end hemofiltration
- S112: remove catheters

## Claims

1. A catheter (1, 101, 102) comprising:
an occlusion member (40) configured for occluding a blood vessel to limit blood flow through the blood vessel,
wherein the catheter (1, 101, 102) is configured to provide a first passage (11) and/or a second passage (21, 21a, 21b, 21c),
wherein the respective passage is configured to provide a connection between an interior of the catheter (1, 101, 102) and an exterior of the catheter (1, 101, 102) within the blood vessel,
wherein the first passage (11) is configured to provide a connection between a first lumen (10) of the catheter (1, 101, 102) and the exterior, and/or
wherein the second passage (21, 21a, 21b, 21c) is configured to provide a connection between a second lumen (20) of the catheter (1, 101, 102) and the exterior.

2. The catheter (1, 101, 102) of claim 1, wherein the first passage (11) is provided on a first side of the occlusion member (40) and wherein the second passage (21, 21a, 21b, 21c) is provided on a second side of the occlusion member (40), such that the occlusion member (40) is positioned between the first passage (11) and the second passage (21, 21a, 21b, 21c) in an extension direction of the catheter (1, 101, 102).

3. The catheter (1, 101, 102) of any one of the preceding claims,
wherein the second passage (21, 21a, 21b, 21c) is positioned distally to the occlusion member (40),
wherein the second passage (21, 21a, 21b, 21c) comprises at least one opening (21a, 21b, 21c), and
wherein a distance between the occlusion member (40) and the at least one opening (21a, 21b, 21c) is at least 13mm.

4. The catheter (1, 101, 102) of any one of the preceding claims, wherein the second passage (21, 21a, 21b, 21c) comprises at least three openings (21a, 21b, 21c) arranged distally to the occlusion member (40), such that a distance between at least some neighboring openings of the at least three openings (21a, 21b, 21c) is the same.

5. The catheter (1, 101, 102) of any one of claims 2 to 4, wherein the shape of at least one of the openings (21a, 21b, 21c) is round or oval with a diameter of at least 4mm.

6. The catheter (1, 101, 102) of any one of the preceding claims, wherein the occlusion member (40) is distanced from a proximal end of the catheter (1, 101, 102) by 5mm to 10mm.

7. The catheter (1, 101, 102) of any one of the preceding claims, wherein the occlusion member (40) is an expandable balloon, wherein an interior of the balloon (40) is connected to an exterior of the catheter (1, 101, 102) outside of the blood vessel via a third lumen (30) of the catheter.

8. The catheter (1, 101, 102) of any one of the preceding claims, wherein the second lumen (20) has a larger cross-sectional area than the first lumen (10).

9. The catheter (1, 101, 102) of any one of the preceding claims, further comprising a connection portion (60) for connecting an interior of at least one of the first lumen (10), the second lumen (20) and/or the third lumen (30) to the exterior of the catheter (1, 101, 102) outside of the blood vessel.

10. An arrangement (100) comprising two catheters (101, 102),
wherein a first catheter (101) is a catheter according to any one of the preceding claims,
wherein the first catheter (101) is configured to be inserted into a blood vessel,
wherein a second catheter (102) is configured to be inserted into the same or another blood vessel, and
wherein the second catheter (102) is a catheter according to any one of the preceding claims, or
wherein the second catheter (102) is a catheter comprising an occlusion member and a passage distal to the occlusion member, wherein the passage is configured to provide a connection between an interior of the second catheter (102) and an exterior of the second catheter (102) within the blood vessel.

11. The arrangement (100) of claim 10, further comprising a tube (105, 106, 107, 108) for connecting each of the catheters (101, 102) to an external device.

12. The arrangement (100) of claim 10 or 11, further comprising a filter element (103).

13. The arrangement (100) of any one of claims 10 to 12, further comprising at least one pneumatic cuff (104a, 104b) configured to limit a blood flow through a body region, when connected to and inflated at the body region.

14. A system (200) comprising:
the arrangement (100) according to any one of claims 10 to 13; and
a pump (300),
wherein the pump (300) is configured to be operatively connectable to or connected to the second lumen of the first catheter (101) and the second lumen of the second catheter (102), and
wherein the pump (300) is configured to circulate a fluid between the first catheter (101) and the second catheter (102).

15. Method of using the arrangement (100) of any one of claims 10 to 13 or the system (200) of claim 14 for treatment of a patient, the method comprising:
i. a step (S101) of inserting the first catheter into a blood vessel of the patient and inserting the second catheter into the same or another blood vessel of the patient; and
ii. a step (S108, S107e) of performing a perfusion circulation between the first catheter (101) and the second catheter (102).
